# EUROPEAN PATENT APPLICATION

(11) **EP 2 450 337 A2**
(43) Date of publication of application: **09.05.2012**
(21) Application number: 11187638.9
(22) Date of filing: 03.11.2011
(51) Int. Cl.: C07C 45/58, C07C 47/42

(54) **Process for making neo-enriched p-menthane compounds**

(30) Priority: 05.11.2010 US 940063
(71) Applicant: Renessenz LLC, Jacksonville, FL 32208-4476 (US)
(72) Inventor: Erman, Mark, B., Atlantic Beach, Florida 32233 (US); Snow, Joe, W., Kingsland, Georgia 31548 (US)
(74) Representative: De Hoop, Eric

(57) **Abstract**

A process for making neo-enriched p-menthane intermediates is disclosed. Lewis acid-catalyzed rearrangement of an oxaspiro compound provides an aldehyde mixture comprising normal (II) and neo (III) p-menthane-3-aldehydes: 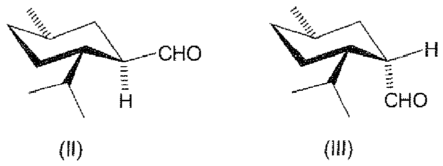 with the neo aldehyde (III) as the major product. The aldehyde mixture is readily oxidized to provide the corresponding carboxylic acids, and the acids are easily converted to a host of neo-enriched p-menthane esters or amides. The esters and amides are valuable as physiological coolants.

## Description

### FIELD OF THE INVENTION

The invention relates to a process for making neo-enriched p-menthane compounds, which are useful intermediates in the flavor and fragrance industry.

### BACKGROUND OF THE INVENTION

Physiological cooling compounds, or coolants, are ubiquitous ingredients in many consumer products, for example chewing gums, toothpastes, mouthwashes, shampoos, lotions and other comestible and cosmetic goods. Chemically activating human cold receptors, they induce a pleasant cooling and refreshing sensation in the consumer, improving the overall appeal of the product.

Known coolants include alcohols, diols, ethers, esters, ketoesters, ketals, carboxamides, and other compounds (see, e.g., Erman, Perfumer & Flavorist 32 (2007) 20, and Leffingwell, "Cooling Ingredients and Their Mechanism of Action" in Handbook of Cosmetic Science and Technology, 3rd Ed. (2009), pp. 661-675.

Among the carboxamide coolants, N-substituted p-menthane-3-carboxamides, exemplified here by "WS-3," "WS-5," and "G-180" are especially important due to their outstanding cooling strength and performance in consumer products. WS-3 and WS-5 were first synthesized by Wilkinson Sword Ltd. (see, e.g., U.S. Pat. No. 4,150,052). G-180 was synthesized much later by Givaudan SA (U.S. Pat. No. 7,414,152). All of these exemplary coolants are derivatives of p-menthane-3-carboxylic acid, also known as WS-1, which was used as a starting material for their syntheses. WS-1 is first converted into its acid chloride WS-1-Cl, typically with thionyl chloride or phosphorous trichloride, and then to an N-substituted amide by reacting the acid chloride with the corresponding amine (Scheme 1). This scheme also shows the synthesis of WS-1 (GB Pat. No. 1,392,907, "Rowsell") by carbonation of menthyl magnesium chloride followed by hydrolysis.

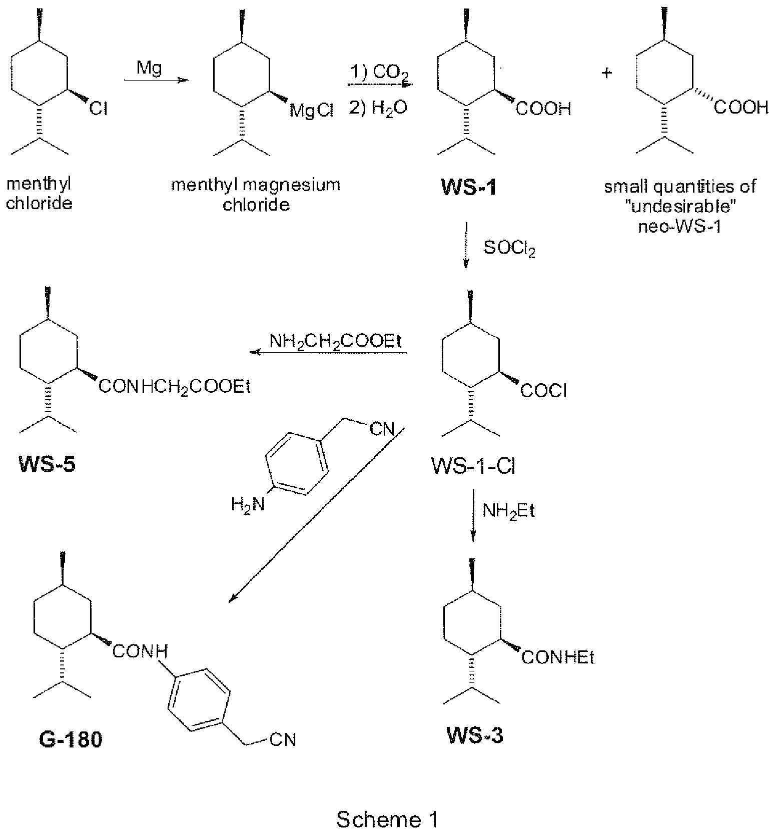

Importantly, Rowsell teaches that in the Grignard reagent preparation step, when 0.5 to 2 M menthyl chloride (or other halide) is used, the resulting WS-1 contains only 70% of the "normal" equatorial isomer and 30% of the "undesirable" axial (neo) isomer. Rowsell also teaches that this proportion can be shifted to favor the normal isomer (95-99% of the normal versus 1-5% of the neo) by increasing the concentration of the menthyl halide to > 4-5 M. Following this pioneering work, all p-menthane-3-carboxamide coolants have been made and sold as practically pure (usually 97+%) normal isomers with the stereochemistry shown in Scheme 1. Analysis of market samples confirms this stereochemistry.

Notably, the stereochemistry reflects that of *l*-menthol - {CAS index name: "cyclohexanol, 5-methyl-2-(1-methylethyl)-, (1R,2S,5R)-"}, which is typically used in industrial syntheses of coolants as a starting material for menthyl chloride (Scheme 2). Note that carbon numbering of menthol and coolants in p-menthane nomenclature differs from the CAS convention, as illustrated in Scheme 2 for structures with function "X."

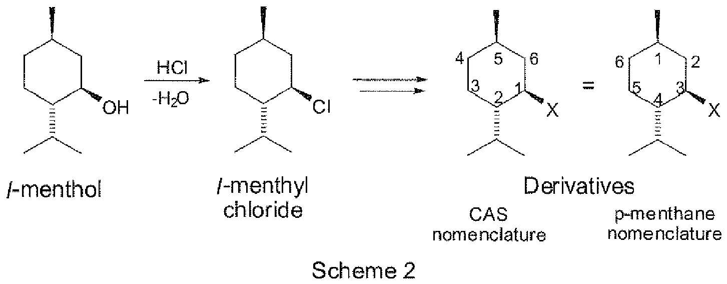

Thus, until recently, all commercial p-menthane-3-carboxamide coolants have been produced from *l*-menthol and sold as isomers with *l*-menthyl stereochemistry. The same is true for ester coolants, including the WS-1 ester derivatives WS-4 and WS-30 (Scheme 3):

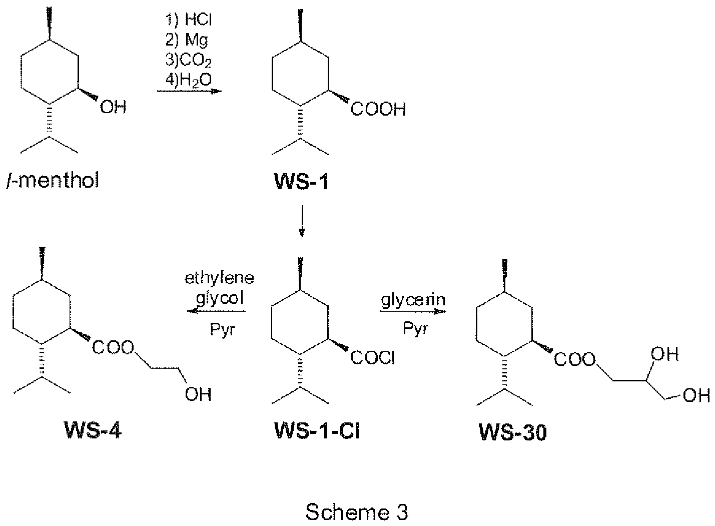

Recently, however, Yelm et al. (U.S. Pat. Appl. 2010/0076080, hereinafter the "the '080 publication") discovered that, at least in some cases, neo (p-menthane-3-axial) isomers of coolants possess superior cooling properties. According to Yelm, the neo isomer [of G-180] unexpectedly has a potent and long-lasting cooling effect. Neo isomers of other derivatives, including menthane carboxy esters and other N-substituted menthane carboxamides were also prepared and found to be useful coolants (see paragraph [0009] of the '080 publication). Yelm also teaches (paragraph [0030]) that mixing the two isomers (normal and neo) of G-180 modulates the negative sensory effects from using normal G-180 alone. Another example of sensory superiority of a neo over a normal isomer is given in the table below paragraph [0137]: the initial cooling rating for neo-WS-5 was 61.9 versus 53.9 for normal WS-5.

Having discovered the value of neo isomers as coolants, Yelm et al. developed synthetic approaches to both normal and neo isomers. For example ('080 publication at paragraph [0025]), normal G-180 can be obtained by reacting WS-1 acid chloride with ammonia to give an unsubstituted amide, followed by arylation with 4-iodophenyl acetonitrile (Scheme 4):

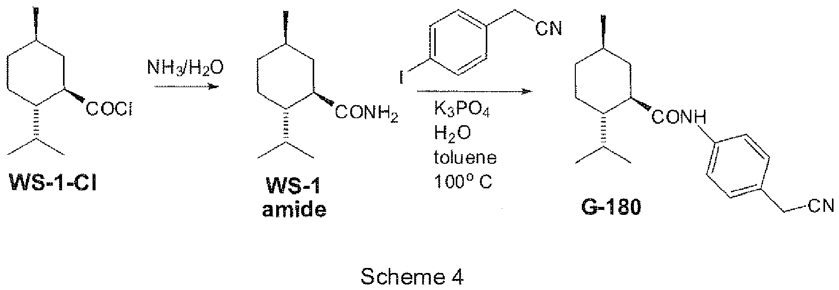

In paragraph [0027], Yelm suggests obtaining p-menthane carboxamides through hydrolysis of the corresponding nitrile, which can be obtained from menthol via menthyl tosylate according to Adolfsson et al. (Tetrahedron: Asymmetry 7 (1996) 1967). In this method, *l*-menthyl tosylate is treated with sodium cyanide in DMSO to give neo-WS-1 nitrile (Scheme 5). Further, the neo-WS-1 nitrile is converted into neo-WS-1 amide by reaction with hydrogen peroxide in DMSO in the presence of NaOH. Finally, the neo-amide is converted into neo-G-180 by coupling with 4-iodophenyl acetonitrile ('080 publication, Example II: A-D).

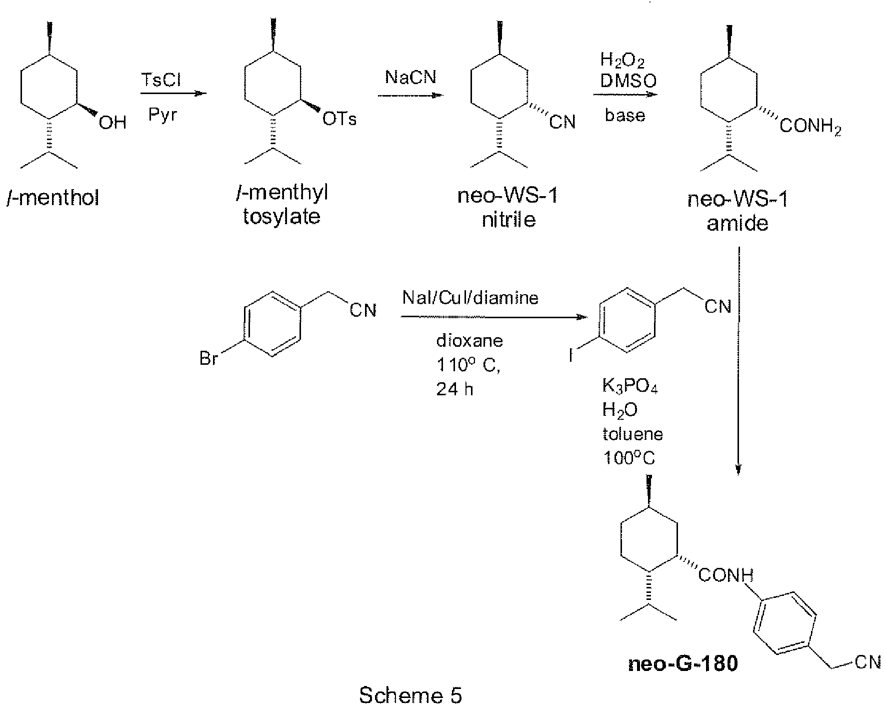

This synthetic approach is unattractive, however, because of the use of highly toxic sodium cyanide and the need to synthesize 4-iodophenyl acetonitrile from the corresponding bromide.

Yelm also teaches (claim 8c, ii) a likely hypothetical process starting with the conversion of *l*-menthol into neo-WS-1 nitrile, where the nitrile is subsequently hydrolyzed using HBr to neo-W5-9. The latter is then converted into either neo-WS-3 or neo-WS-5 by a complex mixture of special reagents as shown on page 5 of the '080 publication and below (Scheme 6).

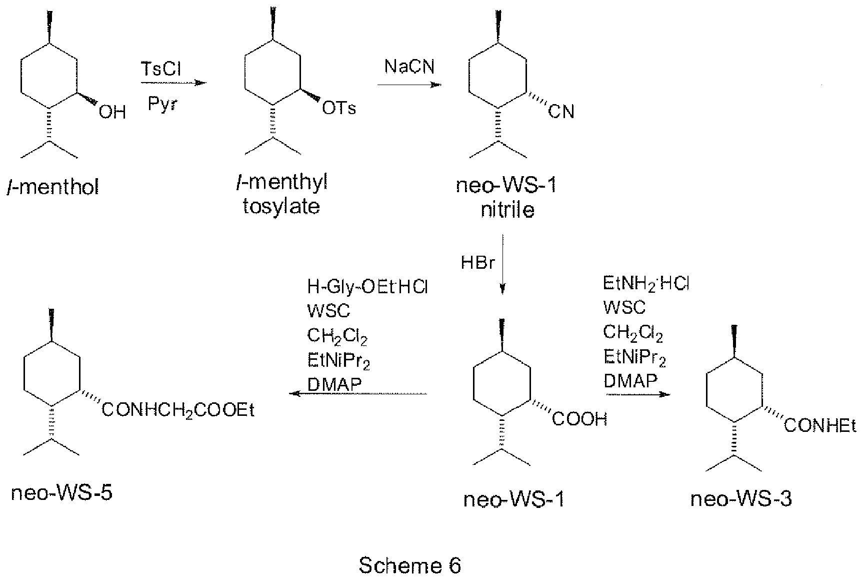

Interestingly, the '080 publication fails to give experimental details, examples, or literature data on the hydrolysis of neo-WS-1 nitrile into neo-WS-1 acid. A paper by Dillner (Organic Preparations and Procedures International 41 (2009) 147) casts doubt on the feasibility of this process. According to Dillner (p. 148, bottom), "all attempts at carrying out the hydrolysis [of neo-WS-1 nitrile] failed." Thus, Dillner used DIBAL-H to convert neo-WS-1 nitrile into neo-WS-1 aldehyde, and then oxidized the aldehyde using Jones reagent (CrO₃/H₂SO₄/acetone) to obtain the desired neo-WS-1 (Scheme 7).

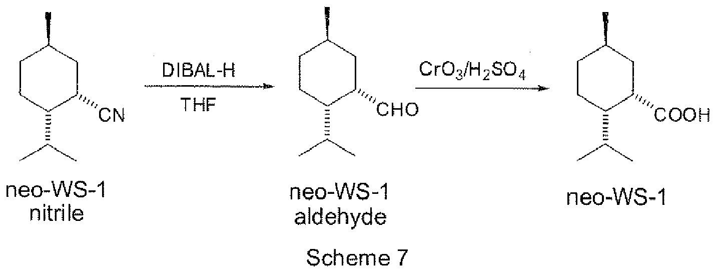

All of the approaches to neo-WS-1 shown above are unattractive mainly because of the use of sodium cyanide. Additionally, the laboratory methods with pyrophoric DIBAL-H and highly toxic chromium(VI) derivatives are preferably avoided in industrial applications.

Thus, a need still exists for a process that would provide neo-WS-1 and its coolant derivatives without employing cyanide chemistry and desirably without using other hazardous reagents.

### SUMMARY OF THE INVENTION

The invention relates to a process for making neo-enriched p-menthane intermediates. The process comprises reacting an oxaspiro compound of formula (I): 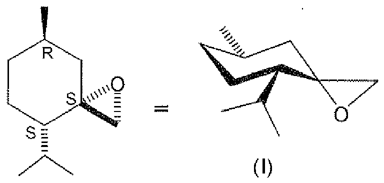 with a Lewis acid to produce an aldehyde mixture comprising normal (II) and neo (III) p-menthane-3-aldehydes: 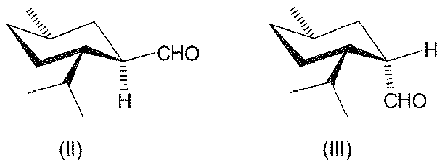 wherein the neo aldehyde (III) is the major product. Surprisingly, Lewis acid-catalyzed ring opening provides more neo than normal isomer.

The aldehyde mixture, which is rich in the neo isomer, is readily oxidized to provide the corresponding carboxylic acids, and the acids are easily converted to a host of neo-enriched p-menthane esters or amides. In each case, the neo configuration is maintained. Thus, the invention provides an improved, cyanide-free route to neo-enriched p-menthanes that have value as physiological coolants.

### DETAILED DESCRIPTION OF THE INVENTION

The inventive process for making neo-enriched p-menthane intermediates begins with oxaspiro compound (I), which is not currently an article of commerce. The oxaspiro compound can be synthesized, however, by any desired route.

In a preferred approach, oxaspiro compound (I) is synthesized from (-)-menthone or its mixtures with (+)-isomenthone (Scheme 8):

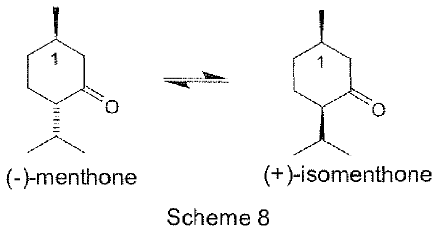

Both menthone stereoisomers occur in many essential oils. A high concentration (sometimes >50%) is found in oils of the *Mentha* genus, which is part of the family of mint oils. The stereoisomers have a strong tendency to interconvert and are, therefore, difficult to obtain in high isomeric purity. Thus, most industrial sources are mixtures of various compositions (see Surburg et al., Common Fragrance and Flavor Materials, 5th Ed. (2006) at p. 63). As used herein, "menthone" means menthone, isomenthone, or their mixtures.

Thus, while known methods of making both normal and neo isomers of WS-1 and coolant derivatives of normal and neo-WS-1 are based on *menthol* as primary starting material, the inventive process uniquely provides, in a preferred aspect, the WS-1 derivatives starting with *menthone.*

In one preferred method (see Duran et al., Tetrahedron: Asymmetry 14 (2003) 2529), menthone is reacted with dimethylsulfoxonium methylide (Scheme 9A), which is prepared *in situ* by reacting trimethylsulfoxonium iodide with sodium hydride in dimethylsulfoxide. Distillation conveniently provides the desired oxaspiro compound (- 80%) as a single enantiomer with equatorial configuration of the newly formed C-C bond. Interestingly, the same result-a single enantiomer-can be obtained from mixtures of (-)-menthone and (+)-isomenthone (Scheme 9B).

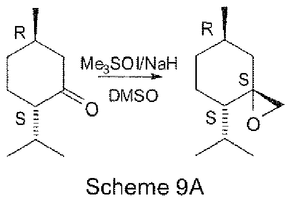

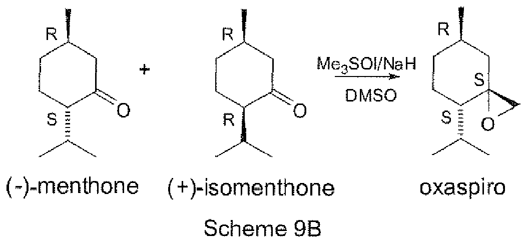

In the inventive process the oxaspiro compound reacts with a Lewis acid to produce an aldehyde mixture comprising normal (II) and neo (III) p-menthane-3-aldehydes: 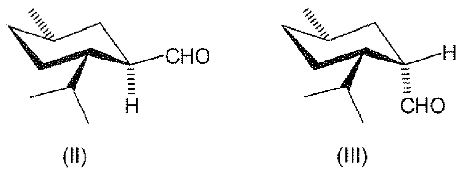 wherein the neo aldehyde (III) is the major product. Preferably, the molar ratio of neo to normal p-menthane-3-aldehydes is greater than 2.

The oxaspiro compound needs no purification (by distillation or otherwise) prior to reacting it with the Lewis acid; instead, crude material obtained from a typical organic workup is preferably used. Because good yields of the aldehydes can be obtained from crude oxaspiro compound, purification is preferably deferred until an easily purified WS-1 acid mixture is generated.

The oxaspiro compound reacts with a Lewis acid. Suitable Lewis acids are well known in the art. They include, for example, zinc chloride, zinc bromide, boron trifluoride (including its etherate complex), lithium perchlorate, iron(III) chloride, tin tetrachloride, and the like. Zinc bromide and zinc chloride are particularly preferred.

Reaction with the Lewis acid is usually performed in the presence of a solvent (hexane, toluene, or the like) under reflux at mild temperatures (e.g., 50-150°C) until the reaction is found by analytical techniques (e.g., gas chromatography) to be reasonably complete. With some Lewis acids, such as boron trifluoride etherate, it may be more desirable to perform the reaction at or below room temperature.

Upon consideration of possible reaction pathways, the skilled person predicts that the C-C bond in the oxaspiro intermediate will retain its equatorial configuration and produce only normal WS-1 aldehyde upon treatment with a Lewis acid. Surprisingly, however, we found that the oxaspiro compound reacts with Lewis acids to give mixtures of products containing both neo and normal WS-1 aldehydes, and predominantly the neo isomer (see Examples 1-8 and Table 1 below). With ZnBr₂ and ZnCl₂, for instance, the WS-1 aldehydes are produced in 40-62% yield (Scheme 10), and the ratio of neo to normal aldehyde is typically ≥ 2.

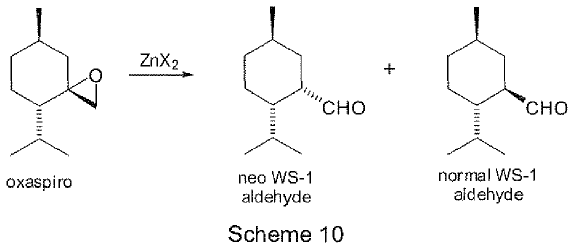

The aldehyde mixture, which comprises normal (II) and neo (III) p-menthane-3-aldehydes, needs no purification, although the skilled person may choose to do so.

More commonly, the aldehyde mixture is converted while still crude to the corresponding mixture of normal and neo p-menthane-3-carboxylic acids. Any desired method for oxidizing the aldehyde mixture to the carboxylic acids can be used. There are many suitable oxidants known to those skilled in the art.

A preferred approach involves aerobic oxidation, a "green" process because the oxidant is the oxygen present in air. Aerobic oxidation can be performed with or without a catalyst. Using a non-catalytic aerobic oxidation of an unpurified aldehyde mixture (see Example 9, below), we obtained a mixture of WS-1 acids (∼ 70% yield) in about the same isomer ratio as in starting aldehydes, i.e., neo:normal ≥ 2 (Scheme 11).

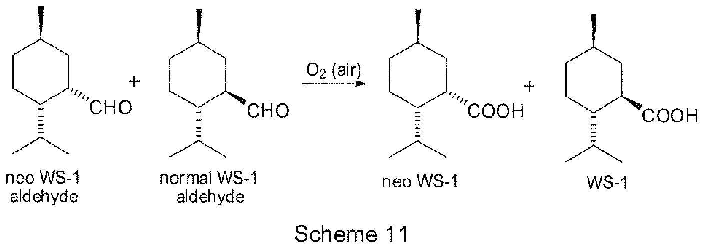

The WS-1 acid mixture is easily purified. Typically, a solution containing the crude acid mixture from oxidation is extracted with dilute aqueous base (sodium hydroxide, potassium bicarbonate, or the like). Separation of phases provides an organic phase containing neutral and/or basic organic impurities, which can be discarded, and an aqueous phase that contains dissolved WS-1 acid salt. After acidifying the aqueous phase, an organic solvent is used to extract the resulting WS-1 acid. Drying and concentration of the organic phase gives the purified WS-1 acid mixture.

The neo-rich WS-1 acid mixture is a versatile intermediate. It is readily converted using well-known methods to a variety of carboxylic acid derivatives, principally esters and amides, that are valuable as physiological coolants. For instance, a neo-enriched mixture of WS-5 isomers is obtained by reacting a WS-1 mixture obtained by the inventive process with a chlorinating agent (thionyl chloride, phosphorus trichloride, or the like) followed by reaction of the resulting acid chloride with glycine ethyl ester. Because the chemistry used to convert WS-1 acids to ester or amide derivatives preserves stereochemical configuration, the derivatives are also neo-enriched. See Examples 10-12, below. Scheme 12 illustrates typical synthetic routes to the commercially important p-menthane amide coolants WS-3, WS-5, and G-180. Neo-isomer enriched *ester* coolants are obtained similarly, as in Scheme 3, by reacting a mixture of neo and normal WS-1 chloroanhydrides with the corresponding alcohols.

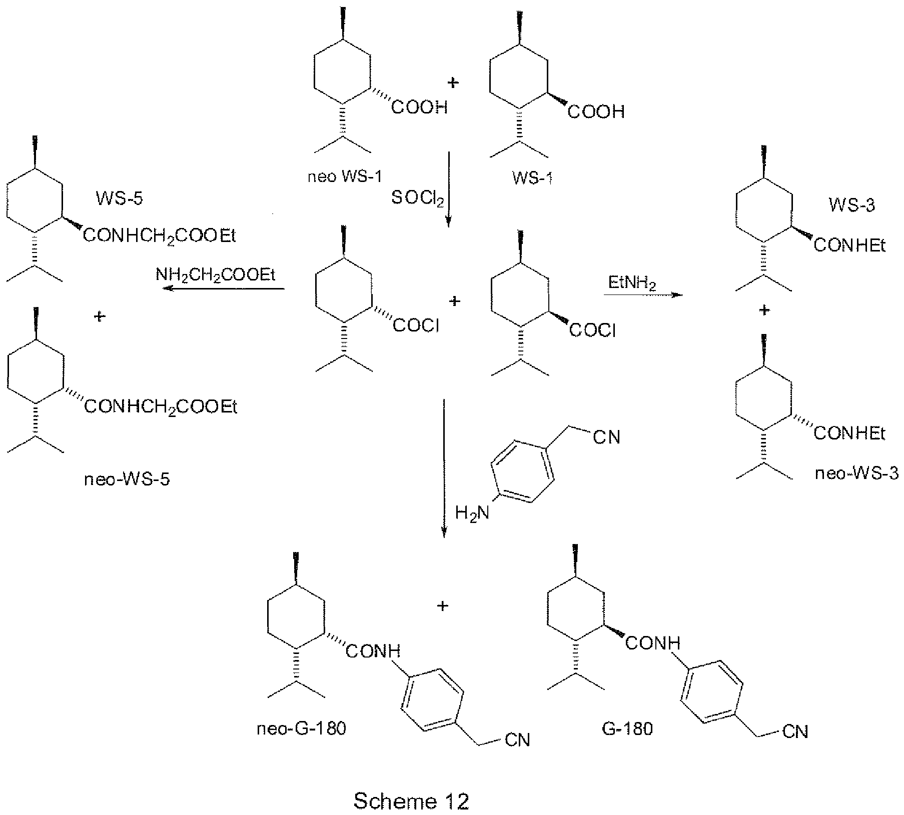

In a preferred aspect of the invention, the ester or amide mixture comprises normal (IV) and neo (V) isomers: 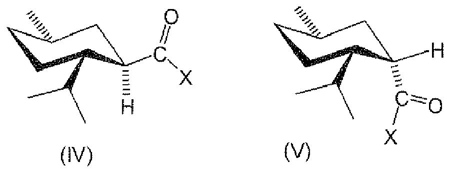 wherein X is OR or NHR¹; R is alkyl, hydroxyalkyl, or alkoxyalkyl; and R¹ is alkyl, hydroxyalkyl, alkoxycarbonylalkyl, aryl, cyanomethylaryl, arylalkyl, or heteroaryl. R is preferably 2-hydroxyethyl or 2,3-dihydroxypropyl. R¹ is preferably ethyl, ethoxycarbonylmethyl, or 4-cyanomethylphenyl.

Neo-enriched p-menthane ester and amide derivatives enabled by the process of the invention are valuable physiological coolants. The coolants are used in many types of consumer products, including comestible items such as chewing gum, chewing tobacco, cigarettes, ice cream, confectionary and drinks, as well as in toiletries and pharmaceutical or cosmetic preparations such as dentifrices, mouthwashes, perfumes, powders, lotions, ointments, oils, creams, sunscreens, shaving creams and aftershaves, shower gels or shampoos.

The following examples merely illustrate the invention. Those skilled in the art will recognize many variations that are within the spirit of the invention and scope of the claims.

### EXAMPLE A

### Preparation of Oxaspiro Compound (I)

The procedure of Duran et al. (Tetrahedron: Asymmetry 14 (2003) 2529) is generally followed. Thus, a 5000-mL, 3-neck flask with N₂ purge and mechanical stirrer is charged with sodium hydride (60% in mineral oil, 50.5 g) and anhydrous dimethylsulfoxide (800 mL). Trimethyl sulfoxonium iodide (231 g, 1.05 mol) is added and the mixture is stirred at room temperature for 3 h. The flask is then placed in an ice bath, cooling the flask to 6°C. A mixture of 83.5% /-menthone/16.5% d-isomenthone (192 mL, 171 g, product of Symrise) is added over 19 min. by addition funnel. Following the addition, the flask temperature is 8°C. The ice bath is removed and the flask is allowed to warm to room temperature. The flask is wrapped in foil to protect the reaction from light. After stirring for 20 h, an analyzed sample shows 13.8 % menthone remaining and 82.4 % of oxaspiro product formed. The flask is cooled to 5°C, and the flask contents are carefully quenched with deionized water (2.6 L). Heptane (450 g) is added to the quenched contents, and the entire mixture is transferred to a 12-L separatory funnel. Deionized water (5 L) is added to the funnel and mixed. The reaction mixture separates overnight. The water layer is drained, and the organic layer is washed with brine. The entire procedure is repeated four more times, and all five organic layers are combined. The combined product is rapidly distilled using an 8" Vigreux column (20 mm Hg). Selected cuts are fractionally re-distilled on a 4' x 1" column (10 mm Hg) to give 556 g of 99% pure oxaspiro intermediate. Yield: 59% from menthone/isomenthone.

### EXAMPLES 1-8

### Lewis Acid-Catalyzed Rearrangement of

### Oxaspiro Compound (I) to WS-1 Aldehydes

Lewis acid (5.0 mmol) is added to a solution of oxaspiro intermediate (0.2 mol) in heptane (100 mL), except for Example 2, where methyl tert-butyl ether is the solvent. The mixture is stirred for the time and at the temperature indicated in Table 1 until complete or almost complete (≤ 2%) disappearance of oxaspiro intermediate. Lewis acids, GC yields of WS-1 aldehydes, and ratios of neo:normal isomers are shown in Table 1.

| Table 1 | | | | | |
|---|---|---|---|---|---|
| Ex. | Lewis acid | Temp., °C | Reaction time, h | Total GC yield, %* | Ratio neo:normal |
| 1 | ZnBr₂ | 101 | 4.2 | 62.4 | 2.44 |
| 2 | ZnBr₂ | 58 | 30 | 21.8 | 2.40 |
| 3 | ZnCl₂ | 80 | 4.0 | 48.5 | 1.06 |
| 4 | ZnCl₂ | 101 | 2.5 | 43.3 | 3.11 |
| 5 | FeCl₃ | 101 | 5.5 | 33.2 | 2.35 |
| 6 | SnCl₄ | 101 | 6.0 | 23.7 | 2.18 |
| 7 | LiClO₄ | 101 | 7.2 | 15.6 | 2.00 |
| 8 | BF₃•Et₂O | 0 to -2 | 2.0 | 5.2 | 1.95 |
| * Not optimized; excludes the solvent component. | | | | | |

### EXAMPLE 9

### Neo Isomer-Enriched WS-1 and WS-1 Acid Chloride

Crude WS-1 aldehydes. Oxaspiro compound (I) (40.0 g, 0.238 mol) is added dropwise over 15 min. to a stirred refluxing (102°C) solution of ZnBr₂ (1.0 g, 0.004 mol) in heptane (230 mL), and the mixture is refluxed and periodically analyzed by GC until the concentration of the oxaspiro intermediate drops below 1% (about 6 h). The reaction is repeated four times, and the products of all five reactions are combined to give a crude mixture (1005 g total) containing by GC 12.46% (124.2 g, 0.74 mol) of WS-1 aldehyde isomers with a neo:normal ratio - 2.3. Yield from (I): 62.2%.

Oxidation to crude WS-1 acids. Air is passed using a ceramic frit bubbler through the stirred crude aldehyde solution (obtained in the previous section) at ambient temperature over 33 h. During the reaction, the solvent (heptane) is maintained in the solution with a dry-ice condenser to supplement a regular condenser with chilled water. The resulting product (1020 g) contains by GC a total of 10.0% (102 g, 0.55 mol) of WS-1 isomers with a neo:normal ratio - 2.1. Theory yield based on starting aldehydes: 74.3%.

Purification of WS-1 acids isomeric mixture. The crude WS-1 acids solution obtained in the preceding section is stirred with NaOH (2300 g of 5% aqueous solution) over 2 h. The mixture is settled and layers are separated. The organic layer contains practically no WS-1 acids (GC). The aqueous layer is acidified with sulfuric acid (1300 g of 10% aq. H₂SO₄) and is then extracted with heptane (600 mL). The layers are separated and aqueous layer is extracted with heptane (480 mL). The heptane extracts are combined, filtered through a thin pad of anhydrous Na₂SO₄ and rotary evaporated to dryness. The residue (95.9 g) is a practically pure (97%) mixture of neo and normal WS-1 acids in a ratio - 2.1.

Neo isomer-enriched WS-1 acid chloride. Phosphorus trichloride (35.9 g, 0.261 mol) is added over 55 min. to a stirred solution of the above-mentioned mixture of neo-WS-1 and normal WS-1 acids (92.9 g) in heptane (130 mL) at 80°C. The mixture is stirred for 3.6 h at 75°C, transferred at this temperature into a separatory funnel, and allowed to cool to ambient temperature. The thick yellow bottom layer (polyphosphorous acid) is thoroughly drained and the remaining heptane solution of neo and normal WS-1 acid chlorides (191.0 g, neo:normal ratio ∼ 2.1 by GC) is used without purification in the following Examples 10-12.

### EXAMPLE 10

### Neo Isomer-Enriched WS-3

The heptane solution of WS-1 acid chlorides (60 g, from Example 9) is added at 20-21°C over 45 min. to a stirred solution of monoethylamine (11.55 g) and sodium carbonate (12 g) in water (180 mL). The mixture is stirred for 3.5 h, and heptane (175 mL) is added. The solid product that forms is separated by filtration and dried to give a mixture of neo and normal WS-3 (14.2 g), total purity 99.0%, neo : normal ratio ∼ 3.1. The filtrate contains two layers. The organic layer is separated, washed with water and rotary evaporated to give an additional mixture of neo and normal WS-3 (12.9 g), total purity 95.7%, neo:normal ratio - 0.8. Combining these two crops and taking into account purities gives a total of 27.0 g of isomeric WS-3 mixture having a neo:normal ratio ∼ 2.1.

### EXAMPLE 11

### Neo Isomer-Enriched WS-5

The heptane solution of WS-1 acid chlorides (60 g, from Example 9) is added at 20-21 °C over 90 min. to a stirred solution of glycine ethyl ester hydrochloride (35.7 g, 0.256 mol) and sodium carbonate (24.7 g) in water (180 mL). The mixture is stirred for 3 h. Heptane (140 mL) is added, and the mixture is heated to 55°C and transferred at this temperature to a separatory funnel. The aqueous layer is drained. The organic layer is washed with hydrochloric acid (95 g of 2% aq. HCl), and washed with water (2 x 190 mL), maintaining the 55°C temperature. The organic layer is refluxed in an apparatus equipped with a Dean-Stark trap to remove residual moisture. The product is transferred into a crystallizer, where it is cooled to ambient temperature. The crystalline mixture of WS-5 isomers is separated by filtration and dried on the filter to give a 98.7% pure isomeric mixture (28.0 g) with a neo:normal ratio ∼2.6. The mother liquor is rotary evaporated to give additional WS-5 isomeric mixture (5.7 g), total purity 97.5%, neo:normal ratio ∼ 0.8. Combining these two crops and taking into account purities gives a total of 32.9 g of an isomeric WS-5 mixture with a neo: normal ratio - 2.14.

### EXAMPLE 12

### Neo Isomer-Enriched G-180

The heptane solution of WS-1 acid chlorides (57.4 g, from Example 9) is added over 30 min. at 20-31°C to a stirred solution of 4-aminophenylacetonitrile (23.3 g, 0.176 mol) in ethyl acetate (160 mL). The mixture is stirred for 1 h and diluted with ethyl acetate (180 mL). After addition of saturated sodium bicarbonate solution (310 mL), the mixture is stirred for 2.5 h and transferred into a separatory funnel. The aqueous layer is drained. The organic layer is filtered through a glass frit and diluted with heptane (1500 mL), which causes precipitation of the product. The crystalline product is separated by filtration and dried on the filter to give 97.9% pure isomeric mixture (29.5 g) with a neo: normal ratio ∼1.4. Evaporation of the mother liquor gives additional G-180 isomeric mixture (15.4 g), total purity 96%, neo:normal ratio -3.0. Combining these two crops and taking into account purities gives a total of 43.7 g of an isomeric G-180 mixture with a neo:normal ratio ∼1.8.

The preceding examples are meant only as illustrations. The following claims define the invention.

## Claims

1. A process for making neo-enriched p-menthane intermediates, comprising reacting an oxaspiro compound of formula (I): 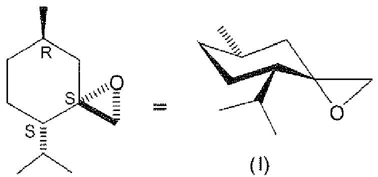 with a Lewis acid to produce an aldehyde mixture comprising normal (II) and neo (III) p-menthane-3-aldehydes: 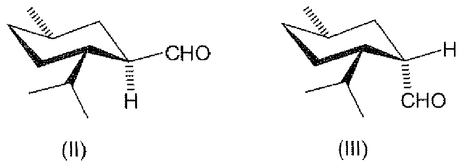 wherein the neo aldehyde (III) is the major product.

2. The process of claim 1 wherein the Lewis acid is selected from the group consisting of zinc chloride, zinc bromide, boron trifluoride, lithium perchlorate, iron trichloride, and tin tetrachloride.

3. The process of claim 1 wherein the molar ratio of neo to normal p-menthane-3-aldehydes is greater than 2.

4. The process of claim **1** wherein the aldehyde mixture is oxidized to give a mixture of the corresponding normal and neo p-menthane-3-carboxylic acids.

5. The process of claim **4** wherein the oxidation is performed aerobically.

6. The process of claim **4** wherein the mixture of p-menthane-3-carboxylic acids is converted to a mixture of esters or amides.

7. The process of claim **6** wherein the ester or amide mixture comprises normal (IV) and neo (V) isomers: 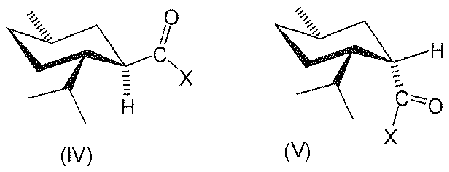 wherein X is OR or NHR¹; R is alkyl, hydroxyalkyl, or alkoxyalkyl; and R¹ is alkyl, hydroxyalkyl, alkoxycarbonylalkyl, aryl, cyanomethylaryl, arylalkyl, or heteroaryl.

8. The process of claim 7 wherein R is 2-hydroxyethyl or 2,3-dihydroxypropyl.

9. The process of claim 7 wherein R¹ is ethyl, ethoxycarbonylmethyl, or 4-cyanomethylphenyl.

10. The process of claim **1** wherein the reaction is performed in the presence of a solvent.

11. An aldehyde mixture made by the process of claim **1**.

12. A neo-enriched carboxylic acid mixture made by the process of claim 4.

13. A neo-enriched ester or amide mixture made by the process of claim **6**.

14. The process of claim **1** wherein the oxaspiro compound (I) is prepared by reacting a sulfur ylide with (-)-menthone, (+)-isomenthane, or a mixture thereof.
